# EUROPEAN PATENT APPLICATION

(11) **EP 3 766 500 A1**
(43) Date of publication of application: **20.01.2021**
(21) Application number: 20184666.4
(22) Date of filing: 08.07.2020
(51) Int. Cl.: A61K 31/70, A61K 31/706, A61K 31/7084, A61K 38/00, A61K 38/06, A61P 25/00, A61P 25/28, A61P 25/30, A61P 39/00

(54) **PHARMACEUTICAL KIT FOR ENHANCED REGENERATION OF THE HUMAN BODY**

(30) Priority: 18.07.2019 EP 19186909
(71) Applicant: Global University Support GmbH, 90443 Nürnberg (DE)
(72) Inventor: KRYZHANOVSKA, Nadiia, 49094 Dnipro (UA)
(74) Representative: Jeck, Anton

(57) **Abstract**

The present invention is related to an articles containing S-adenosyl-methionine, glutathione and NAD+ as combi-nation for the simultaneous, separate or successive administration for use as a medicament.

## Description

The present invention relates in particular to the field of drugs for detoxification, aesthetic medicine, preventive medicine and rehabilitation medicine.

From practice, there is a known drug of the hepatoprotectors group that have a detoxification and antioxidant effect, in particular, the Heptral or Samyr (containing S-adenosyl-methionine 400-500 mg). The drug is used in the treatment of intrahepatic cholestasis (retardation or blocking of the bile's flow) and liver diseases, including acute or chronic cholecystitis, cholangitis; the cirrhotic and pre-cirrhotic conditions; toxic liver damage, including viral, alcohol, drugs (antibiotics, chemotherapy drugs, tuberculosis, antivirals, contraceptives); alcoholic and non-alcoholic fatty liver disease, chronic hepatitis; depressive conditions; withdrawal syndrome, including alcohol.

As it is known the drug Glutathion can be used as a part of the maintenance therapy to reduce the neurological impact of radiation therapy and anticancer chemicals, in which glutathione (GT) must be administered immediately before the start of radiation therapy and before the start of chemotherapy with this drug.

It is known that NAD+ is a clinically substance that supports protein biosynthesis.

Each of the above-mentioned substances can be used separately or in subcombination with other vitamin and / or mineral.

For example, the combination of some of these components with other active agents is disclosed in the invention CN102038867 "Antialcoholism composition and preparation method thereof", which is designed to reduce the absorption of alcohol, speed up metabolism, and for strengthening the stomach and spleen, in which S-adenosyl-methionine (SAM) is combined with other substances (cysteine or methionine; D-glyceric acid or D-glycerate alone or a mixture of D-glyceric acid or g-glycerate, succinic acid or fumaric acid or citric acid or oxalic acid, or malic acid, or a mixture of more than two with citric acid, or citric acid or oxalic acid or malic acid, vitamin B1, vitamin B6 or vitamin B2, or a mixture more than two with vitamin B1, or vitamin B6 or vitamin B2 or coenzyme Q10, L-glutamine).

However, in those compositions the therapeutic effect of a quick detoxification of the body is not fully ensured.

Thus, above mentioned S-adenosyl-methionine, and glutathione are known to be used in separate treatment cycles.

A common drawback of the above-enumerated drugs is e.g. the lack of a single set consisting of isolated from each other active substances for treatment of acute intoxications and antioxidant protection. It should be said that the above-mentioned drugs do not achieve a therapeutic detoxification effect in their usage per se

The **aim** of the present invention is to provide an effective and easy-to-use kit or medicament for enhanced improvement of a patient's state, which will significantly shorten the recovery period e.g. in cases of acute intoxication, such as viral or bacterial infection diseases, (alcohol) intoxication, asthenic syndrome, chronic fatigue syndrome (e.g. from sport training) or drug abuse.

The technical result achieved by using the present invention is, amongst other benefits of the inventive article, combination or kit, an improved recovery period effect, e.g. enhanced restoration of biological detoxification reactions and reaction pathways; direct/imminent elimination through the kit of substances or medicament or it's metabolites; antioxidant protection as well as an accelerated process of recovery of the patient from intoxication side-effects such as e.g. drug abuse and withdrawal symptoms from drug abuse .

Herein a metabolite should be defined as a substance that derive from a chemical or drug that has been introduced into the human or animal body and altered or processed by the metabolism of that human or animal.

The issue is solved with the help of the present invention by the kit containing S-adenosyl-methionine, glutathione and NAD+ as combination for the simultaneous, separate and/or successive/progressive administration. The medicament may be used to treat many conditions including (conditions or symptoms) asthenia, xenobiotic intoxication, convalescence after viral and bacterial infections, posttraumatic state, general and plastic surgery, in particular as eliminating and neutralizing agent of acute intoxications and antioxidant protection agent, and important part of slowing senescence and correction of mitochondrial disfunction.

The conditions for use of this kit may be the premorbid state, morbid state or rehabilitation state.

A condition may be any before mentioned or below mentioned condition or any disease conditions.

A condition may also be a symptom that comes with any of the others condition or sickness. The condition may be particularly related to an increased level of toxins or free radicals in the human body - due to intoxication or cellular senescence.

The first component of S-adenosyl-methionine is preferably used in a dose of 500 mg, the second - glutathione is preferably used in a dose of 600 mg, the third - NAD+ in a dose of 300 mg. The dosages may decrease or increased in individual order.

The dosages may be cut, e.g. Half, a third, a quarter of the before mentioned or more than the before mentioned, e.g.1.5 of the above mentioned, double or triple.

The ratio of the three substances is preferably 1: 1,2 : 0.6. The ratio can vary between 0,5 to 2 for glutathione and between 0.2 to 1.6 for NAD+ each with respect to a ratio of 1 for S-adenosyl-methionine.

The present invention is directed in particular to the following substances and articles as combination of substances which can also be described as combinations, wherein no declaration with respect to the physical separation or combination of the below substances is to be interpreted per se into the word "combination".

The article may contain at least one of the following S-adenosyl-methionine, Glutathione and NAD+ as combination for the simultaneous, separate or successive/progressive administration for use as a medicament.

The article containing S-adenosyl-methionine, Glutathione and NAD+ as combination for the simultaneous, separate or successive/progressive administration for use in neutralizing and/or elimination of acute intoxication, free radical protection (e.g. from chronic diseases and sport training) and geroprotection (protection from premature aging)
Wherein elimination may be understood as direct detoxification via interaction of the inventive article with e.g. toxins as well as support of the human body in the self-restoring process from intoxication

This kit influences the direct elimination of the toxins and at the same time supports the self-restoring process of the human body.

Such type of intoxication may be a result of alcohol-, drug- and stress(-hormone) abuse, as well as the insufficient eliminating phase. Free radicals in the human body are created in the process of detoxification and also need neutralization.

Combination containing S-adenosyl-methionine, glutathione and NAD+ can be prescribed for the simultaneous, separate or successive/progressive administration in the management of insulin resistance.

The management of insulin resistance may result in increased insulin sensitivity.

A decrease of insulin resistance may be recognized e.g. by a decreasing HOMA index (Homeostasis Model Assessment Index) during and after application of the inventive articles/combination or kit.

Combination containing S-adenosyl-methionine, glutathione and NAD+ can be prescribed for the simultaneous, separate or successive/progressive administration for use in the treatment of withdrawal symptoms of drug addicted patients (such as sickness, dizziness, anxiety, muscle debility and sore, insomnia, sweating etc.)
All above stated methods of this kit administration is preferably prescribed at least 5-10 times within 2 weeks for an improved/optimum result.

The present invention also discloses an article/kit for use in the treatment of conditions including asthenia, xenobiotic intoxication, recovery after viral, bacterial and/or fungal infections, wherein the article or kit consists of the following active components which should be used in step by step order - S-adenosyl-methionine glutathione and the last NAD+.

Method for detoxification of acute intoxication, free radical protection and geroprotection wherein S-adenosyl-methionine, glutathione and NAD+ are administered, separately or successively/progressively, preferably in the following order: S-adenosyl-methionine, then glutathione and last NAD+.

The method of the treatment of conditions including asthenia, xenobiotic intoxication, and recovery after viral, bacterial and fungal infections, characterized by administration of the active components isolated from each other in the following sequence: S-adenosyl-methionine, glutathione, NAD+.

The inventive article may also be referred to as a multi component kit or just kit.

The inventive kit comprises or consists of three compounds namely S-adenosyl-methionine, glutathione and NAD+.

According to the present invention, the 3 compounds are preferably introduced into the human body step-by-step 1^{st} S-adenosyl-methionine (SAM), 2^{nd} glutathione (GT) and 3^{rd} NAD+.

According to the order of injections the most preferred way is GT then SAM then NAD+, but all components in parallel is also admitted. The inventive article, as outlined in all variations above, may be used as a recovery kit with detoxification, free radical protection and geroprotection properties, thus the protection from premature aging.

The inventive article has a positive effect on the level of HDL-C (high density lipoprotein cholesterol) and negative (decreasing) effect on the level of LDL-C (low density lipoprotein cholesterol) in the human blood. To see the full potential of changing the LDL-C and HDL-C level one should repeat dosing up to 10 times. The maximum dosage may vary on the intoxication level as well as on the patient's age or weight.

It can be suggested that HDL-C level will be significantly increased after about weeks with 5 dosages per week, with LDL-C level to be decreased consequently.

If NAD+ is applied in combination with SAM and GT, the increase of HDL-C level is achieved faster and more distinct in comparison to NAD+ alone. This increase can be up to 3 times bigger than with NAD+, hence, instead of 5% it goes up to 15% increase of HDL-C.

The combination of NAD+ with SAM and GT lowers LDL-C concentrations in the human blood more effectively in comparison to pure NAD+. In fact, the LDL-C level remains about the same with NAD+ administered in pure form.

The HOMA index is lowered after intake of the inventive article or kit which reflects a decline in insulin resistance. Such a decrease is not observed in administration of NAD+ alone. For the management of Insulin resistant the article or kit should be applied not less than 10 times over 2 weeks.

Accompanying increase in LDH (Lactate dehydrogenase) level up to 30% is noticed after a 10 times per two weeks administration mode of the inventive kit or article, which reflects the process of gluconeogenesis
Although the LDH is an enzyme for lactate to pyruvate transformation the LDH level also reflects a healthy metabolism rather than simply injury of the muscle tissue. The transformation (lactate to pyruvate) is part of the citric cycle which handles the oxidative decomposition of organic material for the purpose of energy gain, wherein for example glucose and fatty acids are consumed. The higher the LDH level the faster ATP is produced in the end, which provides increased endurance of the patient in an (anaerobic) training situation.

Preferably for an increased LDH level of up to 30 % and more, the article or kit should be applied 10 times over a time of at least 2 weeks (thus about 3 times per week).

Overall restoration of the human body is also increased by the inventive article or kit, which is reflected e.g. by shorter recovery times after sport, loss of weight and longer training time in the anaerobic zone, if the treatment is repeated several times (preferably not less than 10 times in a preferred period of at least 2 weeks). In summary, the inventive article, kit or combination of substances yields the effect of initial detoxification and free radical protection followed by a long-term improvement (e.g. detectable by the markers: lipid levels, homocysteine, total antioxidant capacity) and hence geroprotection, which is associated with improved metabolism and toxins elimination.

Glutathione may be regarded as a compound that handles metabolites of the detoxification process conducted by SAM. The toxins elimination is handled by the SAM and glutathione and preferably prepares the body for the direct action of the NAD+ - because NAD+ is the most effective among the components in handling the cholesterol entities.

Thus, SAM and GT prepare the human body for the interaction with NAD+.

The ratio in which the 3 compounds are introduced into the human body are preferably 1: 1,2 : 0.6, wherein 1 can equal 500 mg e.g. for a person of 70 to 80 kg body weight. The ration of the compounds may also vary.

The pure substances should be used by oral (pills, capsules), rectal (suppository), vaginal (suppository), transdermal (ointment, cream, gel, patch) or solution. For injections the solids are tentative dissolved in for example 5 mL of sterile solution (e.g. water) directly prior to application then further dissolved in one hundred millilitres of standard saline.

The 3 substances are preferably applied one after another wherein the application time of each compound can be about 20 minutes. Thus fast application of the 3 compounds one after another is possible.

If repeated application is possible, faster detoxification and improved liver values (general bilirubin, ALT, AST, GGT are decreasing up to 15-30% after first application) are detectable.

The effect of the inventive compound may also help the drug-addicted patients to handle the withdrawal symptoms better. In particular SAM and GT thereby may help with improvement of the patient's state (e.g. asthenia, dizziness, sickness, intoxication), whereby NAD+ may be particularly efficient with the withdrawal symptoms during a long-term treatment (preferably injections should be administered not less than 20 times in at least 4 weeks, thus about 5 injections per week). The inventive kit has in particular the effect of quick detoxification after drug, alcohol, infection and stress abuse plus widespread restoration, due to direct biosynthetic process of new proteins. Another plus NAD+ is highly water-soluble. Consequently, there is no need for modification of NAD+ for effective desorption and dissolving in a suitable liquid for application. For effectiveness biochemical markers can be used e.g. red cells sedimentation rate reducing, homocysteine level decreasing, 8-Oxo-2'-deoxyguanosine level decreasing, glutathione level increasing and MDA level decreasing.

A comparative study with a known compound that contains coenzyme Q10 instead of NAD+ the following effects of the human body were observed:

| | Q10 compound | Inventive Kit |
|---|---|---|
| Total antioxidant capacity | ↑ | ↑ |
| Low weight and protein thiol groups | ↑ | ↑ |
| Glutathione reductase | ↑ | ↑ |
| Lipid peroxides | ↓ | ↓ |
| Homocysteine* | ↓ | ↓ |
| HDL -cholesterol* | No change | ↑ |
| LDL-cholesterol* | No change | ↓ |
| Index HOMA* | No change | ↓ |
| LDH* | No change | ↑ |
| Glutathione peroxidase | ↑ | ↑ |

| | | |
|---|---|---|
| (↓= value decreases, ↑ = value increases) | | |

Long term effects (after intake of at least 20 dosages over at least 4 weeks) exceeded expectations: decrease in waist circumference and body composition (minus 2 kilos on the average), better results of endurance training (higher lactate threshold, longer time in anaerobic zone, better recovery - normalization of the heart rate in 3 minutes after submaximal heart rate peak) were observed.

Lipid lowering and insulin resistance improving action, protein biosynthesis action, increasing pyruvate/lactate ratio (LDH) and alleviation of ATP production (endurance). In sum, they lead to improvement of the body composition and lean body mass increase.

## Claims

1. Kit consisting of S-adenosyl-methionine, glutathione and NAD+ as combination for the simultaneous, separate or successive administration for use as a medicament.

2. Kit containing S-adenosyl-methionine, glutathione and NAD+ as combination for the simultaneous, separate or successive administration for use in elimination of acute intoxication and free radical protection.

3. Kit containing S-adenosyl-methionine, glutathione and NAD+ as combination for the simultaneous, separate or successive administration for use in the treatment of insulin resistance.

4. Kit containing S-adenosyl-methionine, glutathione and NAD+ as combination for the simultaneous, separate or successive administration for use in the treatment of withdrawal symptoms of drug addicted patients.

5. Kit for use in the treatment of conditions consisting of asthenia, xenobiotic intoxication, convalescence after viral infections, senescence associated states, mitochondrial disfunction, posttraumatic state, general and plastic surgery, asthenic syndrome and/or chronic fatigue syndrome in that it consists of the following active components stored isolated from each other, which active components are applied together or sequentially in the following sequence: S-adenosyl-methionine, glutathione, NAD+.

6. Kit according to any of claim 1 to 5 **characterized in that** S-adenosyl-methionine, glutathione, NAD+ are applied in a ratio of 1 : 1,2 : 0,6.

7. Kit according to any of claim 1 to 6 **characterized in that** components are applied as a single or multiple saline solutions, oral intake, transrectal intake, transvaginal intake, transdermal application, transmucosal application and injections of saline solutions.

8. Kit according to any of claim 1 to 7 **characterized in that** the articles are applied at least 5 times in one week, preferably 10 times within 2 weeks.
